# EUROPEAN PATENT SPECIFICATION

(11) **EP 1 569 546 B1**
(45) Date of publication and mention of the grant of the patent: **06.06.2007**
(21) Application number: 03775614.5
(22) Date of filing: 26.11.2003
(51) Int. Cl.: A61B 3/028, G02B 3/14

(54) **EYE TESTING**
AUGENUNTERSUCHUNG
EXAMEN DE LA VUE

(30) Priority: 03.12.2002 EP 02080061
(43) Date of publication of application: 07.09.2005
(73) Proprietor: Koninklijke Philips Electronics N.V., 5621 BA Eindhoven (NL)
(72) Inventor: KUIPER, Stein, NL-5656 AA Eindhoven (NL); WOLTERINK, Edwin, M., NL-5656 AA Eindhoven (NL)
(74) Representative: van der Veer, Johannis Leendert
(86) International application number: PCT/IB2003/005434
(87) International publication number: WO 2004/049927

(56) References cited:
- WO-A-02/09579
- DE-A- 4 217 853
- US-A- 3 880 502
- US-B1- 6 369 954

## Description

The present invention relates to apparatus for, and a method of, testing the eyes of a patient for eye deviation.

An eye, for example that of a human, when working perfectly is capable of receiving light rays from the surroundings and by focusing these on the retina of the eye. The brain is able to interpret the information as a clear visible image. It is common however for an eye to function imperfectly thus resulting in an incorrectly viewed image. Two common conditions of the eye which affect the ability to view an image are termed "spherical" eye deviation and "astigmatic" eye deviation. Spherical eye deviation occurs when the lens of the eye focuses the incoming light rays at a focal point which is not on the eye's retina and therefore the resulting image viewed by the brain appears out of focus. Astigmatic eye deviation occurs when the lens of the eye becomes anamorphic and focuses the incoming light rays at two focal lines which are generally orthogonal and axially separated. The resulting image viewed by the brain generally appears blurred or distorted. A patient's eye may exhibit one of, or a combination of, these two common eye deviations.

For both these conditions it is possible for corrective lenses in the form of, for example, spectacles or contact lenses to correct the angle of approach of incoming light rays to the eye so that they are correctly focused on the retina. As a result, the image viewed by the brain is corrected.

In order to determine the exact refractive characteristics of the corrective lenses needed for the patient's eye deviation, a test is performed by a qualified person, for example an ophthalmic optician. Current methods for testing eye deviation involve individually placing a large variety of lenses of differing refractive characteristics in front of the eye and asking the patient to view a distant testing object, in the form for example of a chart displaying black alphabetical letters of varying sizes, and comment whether the vision is better or worse. The lenses used for testing differ in strength between each other by a certain quantitative step in focal strength, for example 0.25 Dioptres, or astigmatism. Such a method for testing eye deviation is relatively elaborate and time-consuming.

Document WO 02/09579 describes an ophthalmic apparatus for the testing of eye deviation of a patient's eyes, comprising a variable lens, control means for controlling the refractive characteristics of said variable lens and output means for outputting a data value indicative of a measured eye deviation for the patient.

It is an object of the present invention to provide improved ophthalmic apparatus for the testing of eye deviation of a patient's eyes.

A further object of the present invention is to provide an improved method of testing the eye deviation of a patient's eyes.

According to the present invention there is provided ophthalmic apparatus for the testing of eye deviation of a patient's eyes, said apparatus comprising a variable lens having refractive characteristics which cause alterations in direction of rays of light passing through the lens along predetermined incident paths, means for controlling the refractive characteristics of said variable lens, during the measurement of a patient's eye deviation, and means for outputting a data value indicative of a measured eye deviation for the patient, whereby said variable lens comprises a meniscus, wherein the meniscus separates a layer of a first fluid and a layer of a different, second fluid, and a plurality of electrodes spaced about said optical axis, wherein said control means is adapted to achieve different meniscus shapes by a variation of a pattern of voltages applied across said plurality of electrodes, wherein said meniscus shapes are lens shapes having variable refractive characteristics and including at least approximately spherical or aspherical and at least approximately anamorphic lens shapes.

Furthermore, according to the present invention there is provided a method of testing the eye deviation of a patient's eyes according to claim 13.

Using the present invention, a test procedure can be conducted more efficiently than the conventional method as the variable lens of the present invention eliminates the need to constantly swap the lens through which the patient views a test chart.. A more accurate result can also be achieved than the traditional method as configurations of the variable lens can be obtained which differ between each other by only relatively small quantitative steps of, or continually varying, refractive characteristics. The variable lens of the present invention is capable of achieving non-rotationally symmetric lens configurations. Such a non-rotationally symmetric lens configuration can be rotated about an optical axis of the lens in order to achieve a desired rotational position for the testing of the patient's eye deviation.

In a preferred embodiment, a fluid meniscus lens is used, allowing effective variation of the refractive characteristics of the lens with considerable accuracy and variability. It is noted that a fluid meniscus lens is described in international patent applications WO 99/18456 and WO 00/58763, however the prior art lenses are proposed for use in relation to optoelectronic systems and endoscopy.

The apparatus preferably include a configuration of electrodes for altering the configuration of the fluid meniscus lens. In preferred embodiments, the electrode configuration comprises one or more pairs of electrodes on opposite sides of said optical axis. In this arrangement, the electrodes can be driven to provide anamorphic lens configurations for measuring a patient's eye astigmatism.

The apparatus preferably comprises a testing object comprising ophthalmic indicia for viewing by a patient during the testing of eye deviation. The patient can then view the testing object, in a manner similar to conventional eye testing, to determine whether the lens configuration is providing an acceptable degree of compensation for any eye deviation. At this point, one or more data values provided by the ophthalmic apparatus may be used to produce an ophthalmic prescription for use in providing corrective spectacles or contact lenses for the patient.

Further features and advantages of the invention will become apparent from the following description of preferred embodiments of the invention, given by way of example only and made with reference to the accompanying drawings.
Figs. 1 to 3 show a simplified cross-section of a variable lens in various focusing stages in accordance with an embodiment of the present invention;
Fig. 4 is a schematic diagram of an embodiment of the present invention for testing eyes;
Fig. 5 a shows an electrode configuration for use in a variable lens in accordance with an embodiment of the present invention;
Fig. 5b shows an alternative electrode configuration for use in a variable lens according to an embodiment of the invention;
Fig. 5c shows a further alternative electrode configuration for use in a variable lens according to an embodiment of the invention; and
Fig. 6 shows a graphical representation of voltages applied across an electrode configuration according to an embodiment of the invention.

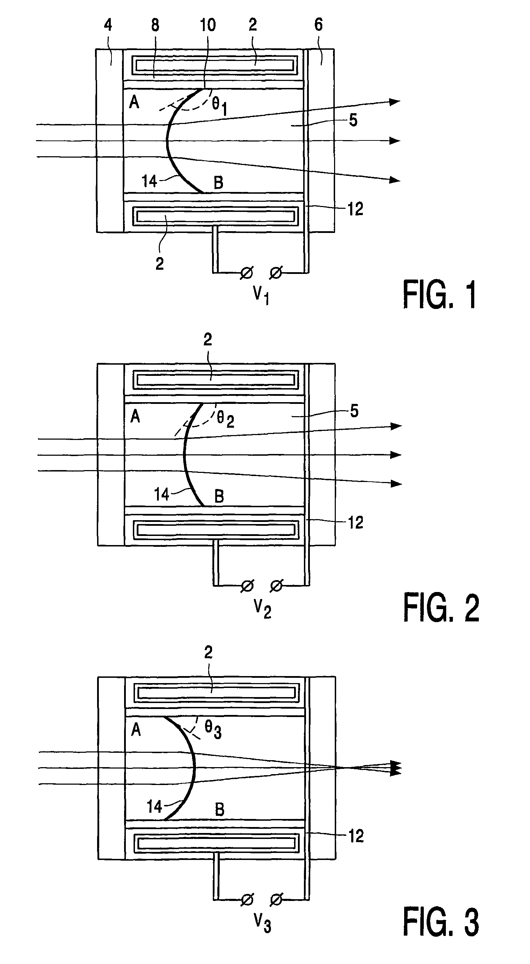

Figs. 1 to 3 are schematic cross-sections which show a variable lens according to one embodiment of the present invention. A description of the structure and function of the lens follows. The lens in this embodiment is a variable focus lens comprising a cylindrical sidewall electrode 2 forming a tube, sealed by means of a transparent front element 4 and a transparent back element 6 to form a fluid chamber 5 containing two fluids.

In this embodiment the two fluids consist of two non-miscible liquids in the form of an non-conducting non-polar first liquid A, such as a silicone oil or an alkane, referred to herein further as "the oil", and an electrically conducting polar second liquid B, such as water containing a salt solution. The two liquids are preferably arranged to have an equal density, so that the lens functions independently of orientation, i.e. without dependence on gravitational effects between the two liquids. This may be achieved by appropriate selection of the first or second liquid constituents.

Depending on the choice of the oil used, the refractive index of the oil may vary between 1.25 and 1.60. Likewise, depending on the amount of salt added, the salt solution may vary in refractive index between 1.33 and 1.48. The fluids in this embodiment are selected such that the first fluid A has a higher refractive index than the second fluid B.

The sidewall electrode 2 is formed from a metallic material and is coated by an insulating layer 8, formed for example of parylene. The insulating layer is coated with a fluid contact layer 10, which reduces the hysteresis in the contact angle of the meniscus with the cylindrical wall of the fluid chamber. The fluid contact layer is preferably formed from an amorphous fluorocarbon such as Teflon^{™} AF1600 produced by DuPont^{™}. The wettability of the fluid contact layer by the second fluid is substantially equal on both sides of the intersection of the meniscus 14 with the fluid contact layer 10 when no voltage is applied.

An annular endwall electrode 12 is arranged at one end of the fluid chamber, in this case, adjacent the back element 6. The endwall electrode 12 is arranged with at least one part in the fluid chamber such that the electrode acts on the second fluid B.

The two fluids A and B in this embodiment are non-miscible liquids so as to tend to separate into two fluid bodies separated by a meniscus 14. When no voltage is applied between the sidewall and endwall electrodes, the fluid contact layer has a higher wettability with respect to the first fluid A than the second fluid B. Due to electrowetting, the wettability by the second fluid B varies under the application of a voltage between the sidewall electrode and the endwall electrode, which tends to change the contact angle of the meniscus at the three phase line (the line of contact between the fluid contact layer 10 and the two liquids A and B). The shape of the meniscus is thus variable in dependence on the voltages applied.

Referring now to Fig. 1, when a low voltage V₁, e.g. between 0 V and 20 V, is applied between the electrodes the meniscus adopts a first concave meniscus shape. In this configuration, the initial contact angle θ₁ between the meniscus and the fluid contact layer 10, measured in the fluid B, is for example approximately 140°. Due to the higher refractive index of the first fluid A than the second fluid B, the lens formed by the meniscus, here called fluid meniscus lens, has a relatively high negative power in this configuration.

To reduce the concavity of the meniscus shape, a higher magnitude of voltage is applied between the first and second electrodes. Referring now to Fig. 2, when an intermediate voltage V₂, e.g. between 20 V and 150 V, depending on the thickness of the insulating layer, is applied between the electrodes the meniscus adopts a second concave meniscus shape having a radius of curvature increased in comparison with the meniscus in Fig. 1. In this configuration, the intermediate contact angle θ₂ between the first fluid A and the fluid contact layer 10 is for example approximately 100°. Due to the higher refractive index of the first fluid A than the second fluid B, the fluid meniscus lens in this configuration has a relatively low negative power.

To produce a convex meniscus shape, a yet higher magnitude of voltage is applied between the first and second electrodes. Referring now to Fig. 3, when a relatively high voltage V₃, e.g. 150 V to 200 V, is applied between the electrodes the meniscus adopts a meniscus shape in which the meniscus is convex. In this configuration, the maximum contact angle *θ*₃ between the first fluid A and the fluid contact layer 10 is for example approximately 60°. Due to the higher refractive index of the first fluid A than the second fluid B, the fluid meniscus lens in this configuration has a positive power.

Thus, through variation of the voltages applied, various different approximately spherical, including aspherical, lens shapes can be generated.

Although the fluid A has a higher refractive index than fluid B in the above example, the fluid A may also have a lower refractive index than fluid B. For example, the fluid A may be a (per)fluorinated oil, which has a lower refractive index than water. In this case the amorphous fluoropolymer layer is preferably not used, because it might dissolve in fluorinated oils. An alternative fluid contact layer is e.g. a paraffin coating.

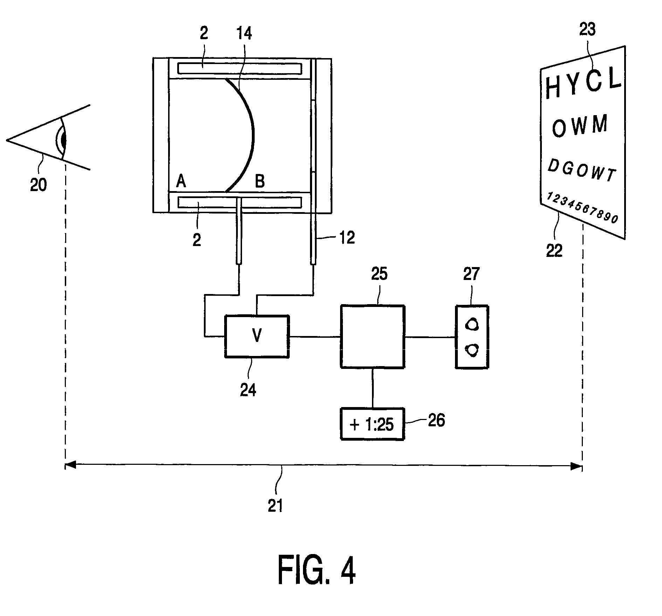

Fig. 4 shows a schematic diagram of eye testing apparatus according to an embodiment of the present invention. A patient, whose eyes 20 are to be tested for eye deviation, is positioned at a predetermined distance 21 from a testing object. The testing object presents media used for eye testing to the eyes of the patient. For example the testing object may be a test chart 22 displaying consecutive rows of ophthalmic indicia in the form of black alphabetical letters 23 against a white background. The font size of the letters of each consecutive row decreases. The size of at least some of the rows of alphabetical letters is determined using data values including the predetermined distance 21, between the eyes of the patient 20 and the test chart 22 and a predetermined threshold of acceptable eye deviation.

A variable focus lens, such as that previously described using Figs. 1 to 3, is positioned at a predetermined distance between the eyes 20 to be tested of the patient and the test chart 22, for example by means of a head-mounted frame. Such positioning causes the optical axis of the variable focus lens to lie along the path of light between the test object 22 and the eyes 20 to be tested. The variable focus lens in this embodiment includes a voltage control unit 24, for example a variable resistance element, connected between the sidewall electrode 2 and the endwall electrode 12. An electronic control unit 25 is connected to the voltage control unit 24. A manual control unit 27 containing one or more manual control elements for example rotatable knobs, for altering characteristics of the lens, is connected to the electronic control unit 25. Adjusting a 'focal power' control on the manual control unit 27 allows the voltages applied across electrodes 2 and 12 to be varied continually or step-wise altered, thus in turn varying the focal power of the lens, as previously described. A data display 26 connected to the electronic control unit gives a readout of one or more refractive characteristics, such as the focal power of the lens at the present voltages applied. The display may be a dedicated element, such as a liquid crystal display (LCD) or may form part of a data processing system, such as the display of a general purpose computer. To achieve this, the display is calibrated to provide the correct conversion between voltages applied and focal power. The focal power of the lens is preferably shown in focal power units of Dioptres (herein abbreviated to "D") corresponding to the currently applied voltages.

The eye deviation test is performed by a suitably qualified person such as an ophthalmic optician. It is envisaged that several methods could be used to test each individual eye for eye deviation. One example involves initially testing each eye individually for eye deviation whilst the other patient's eye is temporarily shaded from viewing. Once both eyes have been tested individually the combined viewing ability of both eyes may be tested together. During the test the ophthalmic optician requests the patient whose eyes 20 are being tested to provide feedback based upon their ability to view clearly the test chart 22. In this example the patient is asked to view and read the letters from different rows of the test chart 22. The variable focus lens may initially be set to a focal power value of 0 D or to a focal power matching that detailed on a current optical prescription of the patient.

Feedback given by the patient, for example by means of speech, based upon their ability to view test chart 22, is interpreted by the ophthalmic optician performing the eye test. Upon interpreting this feedback the ophthalmic optician varies the focal power of the variable focus lens as necessary using the manual control unit 27 until the patient is able to view the test chart 22 at a predetermined threshold of ability.

In an envisaged alternative to this embodiment, the patient varies the focal power of the variable focus lens using the manual control unit 27 based upon their own ability to view the test chart 22. This enables the required focal power of the variable focus lens required for the patient to achieve the predetermined threshold of viewing ability to be determined more quickly and/or accurately.

The display 26 shows the value of focal power of the variable focus lens needed for the patient to achieve the predetermined threshold of viewing ability. This value is recorded by the ophthalmic optician and forms part of the patient's new optical lens prescription. A positive recorded value of focal power corresponds to a "long-sighted" eye deviation condition. A negative recorded value of focal power corresponds to a "short-sighted" eye deviation condition.

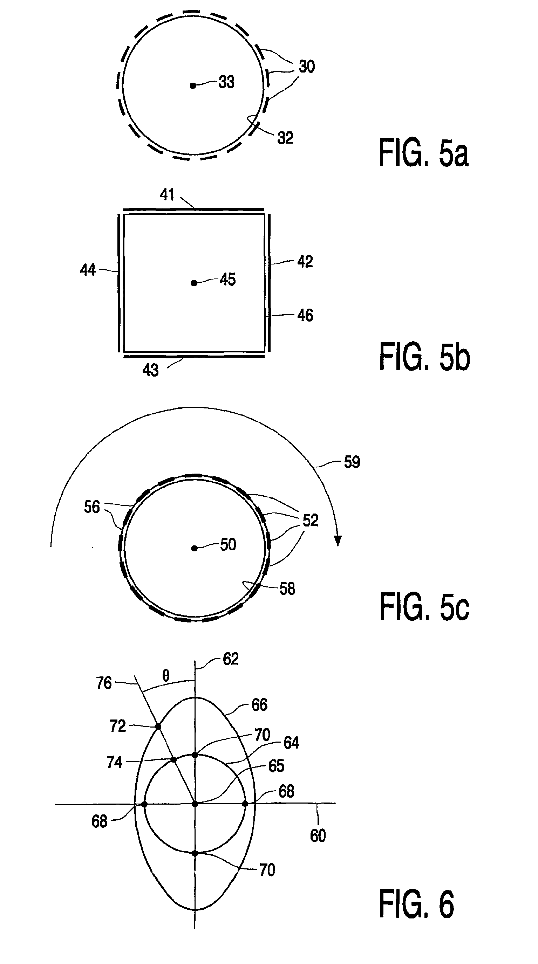

Fig. 5 a, being a cross-section taken perpendicular to the optical axis of the lens, shows an alternative electrode configuration for use in a variable lens capable of producing anamorphic lens shapes. In this embodiment the lens is capable of generating variable focal powers and/or variable amounts and types of astigmatism. A plurality of individual sidewall segment electrodes 30, each rectangular in side view (not shown) and arranged side-by-side and spaced about the optical axis 33, form a cylindrical enclosure.

In this embodiment, the manual control unit 27 possesses separate controls for varying each of the focal power, cylindrical value and cylindrical axis. The remaining characteristics of the apparatus may be as described in relation to the previous embodiments. The segment electrodes are formed from a metallic material. The cylindrical inner surface described by the arrangement of segment electrodes is covered with a continuous, uniform thickness, insulating, fluid contact layer 32 formed for example of parylene. Each individual segment electrode is also insulated with respect to the adjacent electrodes.

Referring now to both Figs. 1 and 5a, and with substitution of the sidewall electrode 2 with the alternative electrode configuration of a plurality of segment electrodes, an independently varying voltage can be applied between an endwall electrode similar to annular electrode 12 and each individual segment electrode 30. In this embodiment of the invention, the use of the focal power and cylindrical value controls on the manual control unit 2 provided are capable, via the electronic control unit 25 and the voltage control unit 24, of controlling each individual applied voltage differently according to a desired voltage pattern. Preferably, the electrodes are arranged in pairs on opposite sides of the optical axis 33 and are provided with the same levels of applied voltage, and the voltages applied vary gradually between electrodes in the direction of the lens circumference. The average applied voltage is related to the focal power whilst the largest voltage variation is related to the cylindrical value. The pattern of voltages applied on the segment electrodes 30 can be electronically rotated about the optical axis 33 using the cylindrical axis control of the manual control unit 27, which in turn alters the orientation of the lens astigmatism about the optical axis. This enables the correct angular position of the cylindrical axis of the anamorphic lens to be obtained.

Using this embodiment of the invention, with the variable lens of Fig. 5a, it is possible for an ophthalmic optician to also test the astigmatic eye deviation of the eyes of a patient being tested.

Furthermore, by controlling the voltages to generate a constant voltage difference between each individual segment electrode 30 and the endwall electrode, the spherical eye deviation of the eyes can be examined in a similar manner to that described above.

Through different combinations of individual voltages applied across electrodes it is possible to obtain various meniscus shapes including those of approximately spherical, and anamorphic, e.g. approximately cylindrical and approximately spherocylindrical, natures.

Fig. 6 shows a graphical representation of relative values of voltages in patterns of voltages applied to produce anamorphic lens shapes. Any relative value of voltage applied at an electrode can be determined by taking the radial distance between the two lines 64, 66 at the appropriate angular position corresponding to the angular location of the center of the electrode about the optical axis 65. In the following, the angular positions corresponds the position about the circumference of the arrangement of segment electrodes described using Fig. 5a. The graphical representation shows a plot on perpendicular axes of this variation of voltages corresponding to a cross-sectional view perpendicular to an optical axis of the fluid meniscus lens. The graphical representation shows a first axis 60 and a second axis 62, arranged perpendicular to each other. The first axis 60 corresponds to a cylindrical axis of the meniscus shape. The circular circumferential line 64 is used to represent all the possible locations of the centers of the segment electrodes 30 (not shown in Fig. 6) about the optical axis. Locations corresponding to the centers of two pairs of the rectangular segment electrodes, perpendicular to each other, are shown; 68 and 70 respectively, in this case lying along axes 60 and 62 respectively.

Applied voltage line 66 shows relatively the applied value of voltage corresponding to a point on the circumferential line 64 of the electrode arrangement. In the representation, the radial distance between a point on the applied voltage line 66 and the corresponding point on the circumferential line 64 represents the relative applied voltage, the common radial line lying at a specific angle from one of either axis 60 or 62. By way of example, this is illustrated in Fig. 6 wherein label 72 shows the point on the applied voltage line 66 and label 74 shows the corresponding point on the circumferential line 64. Both of these points lie along the common radial line 76 at angle θ from, in this case, axis 62.
The greater the radial distance between the point on the applied voltage line 66 and the corresponding point on the circumferential line 64, the greater the relative applied voltage. For example, as Fig. 6 shows, a relatively high voltage is applied across segment electrode pair represented by locations 70, whereas a relatively low voltage is applied across segment electrode pair represented by locations 68. The voltages applied across each respective intermediate segment electrode 30, arranged between a member of the segment electrode pair represented by locations 70 and a member of the segment electrode pair represented by locations 68, decreases gradually.

In this embodiment the width of each segment electrode 30 is less than half, preferably less than one eighth, of the internal diameter of the cylindrical arrangement of electrodes. This involves the use of sufficient electrodes, preferably sixteen or above, to reduce observation at the center of the fluid meniscus lens of significant effects caused by discrete steps of meniscus contact angle between the cylindrical walls of the fluid chamber.

To test the eyes of the patient for astigmatic eye deviation a similar method is used to that used for spherical eye deviation testing. For best results, it is preferable for the nature of media presented on the test chart 22 to be specific to astigmatic eye deviation testing. As when testing for spherical eye deviation the ophthalmic optician performing the test or the patient themself varies the voltages applied using the appropriate controls on the manual control unit 27.

In this case, the individual voltages applied across the endwall electrode and each individual segment electrode 30 are varied differently, under the control of the electronic control unit 25 to produce voltage patterns for different desired anamorphic lens shapes. Thus the fluid meniscus lens shape is varied by the ophthalmic optician or the patient based upon feedback information given by the patient on their ability of viewing the test chart 22. Once the viewing ability of the patient is at a predetermined threshold a record of the output values for astigmatic eye deviation is made. These values are displayed by the display 26. The display 26 in this embodiment of the invention is capable of converting applied electrode voltage values for both spherical and astigmatic eye deviation tests and yielding relevant refractive characteristic data for the eye being tested. Recorded values for the spherical and astigmatic eye deviation are then used to generate a patient's new optical lens prescription.

The data provided by the patient's optical prescription may subsequently be used to manufacture corrective lenses, for example spectacles or contact lenses, which allow the patient to maintain the required threshold of viewing ability.

Fig. 5b, being a cross-section taken perpendicular to the optical axis of the lens, shows a simplified alternative electrode configuration for producing anamorphic fluid meniscus lens shapes. Four rectangular segment electrodes 41, 42, 43, 44 are spaced about the optical axis 45 of the lens in a square formation with their longitudinal edges parallel, thus forming a square enclosure. The inner surface of the segment electrodes is covered with a continuous, uniform thickness, electrically insulating, fluid contact layer 46, formed for example of parylene.

Referring now to both Figs. 1 and 5b and with substitution of the sidewall electrode 2 by the alternative construction of four segment electrodes, a voltage can be applied between a single segment electrode 41, 42, 43 or 44 and an endwall electrode similar to the annular electrode 12 in the first embodiment. Through combinations of different voltages applied to the segment electrodes 41, 42, 43 or 44, an anamorphic fluid meniscus lens shape which is approximately cylindrical or spherocylindrical can be achieved with a different contact angle between each individual segment electrode wall and the fluid meniscus lens. As in the previous embodiment, the pattern of voltages applied is controlled using the controls of the manual control unit 27 via the electronic control unit 25 and the voltage control unit 24.

In this embodiment, a lens rotation mechanism is provided and the cylindrical axis control of the manual control unit 27 results in automatic mechanical rotation of the variable lens about the optical axis 45. This enables the variable lens to be correctly positioned angularly for correct astigmatic reading to be obtained.

By connecting the voltages applied across opposite segment electrodes as pairs, in other words electrodes 41 and 43 as a pair, and electrodes 42 and 44 as a pair, and applying the voltages across each connected electrode pair and the endwall electrode 12 similarly or differently, it is possible to obtain spherical, cylindrical or spherocylindrical lens shapes.

Fig. 6 as described in the previous embodiment illustrates an example of a pattern of applied electrode voltages. In this particular embodiment the two pairs of segment electrodes of the configuration correspond to the fig. labels 68 and 70. The use of this alternative electrode configuration in this embodiment of the invention allows both spherical and astigmatic eye deviation to be tested in a manner similar to that detailed in the previous embodiment. The lens configuration may be used to measure both, or only one of spherical eye deviation and astigmatic eye deviation.

Fig. 5c, being a cross-section taken perpendicular to the optical axis of the lens, shows a further alternative electrode configuration for producing anamorphic lens shapes. This electrode configuration is used to achieve lens shapes with reduced optical aberrations.

As in the previously described alternative electrode configurations of previous embodiments of the present invention, illustrated with Figs. 5a and 5b, the segment electrodes 52 in this embodiment are formed from a conductive metallic material. The inner surface of the enclosure described by the arrangement of electrodes is covered with a continuous, uniform thickness, electrically insulating, fluid contact layer 58 formed for example ofparylene. The segment electrodes 52 are spaced about the optical axis 50 with their longitudinal edges parallel to define an enclosure. In this example, individual segment electrodes 52 arranged to form a cylindrical enclosure about the optical axis 50. The longitudinal edge of each individual electrode is connected to the parallel and adjacent longitudinal edge of the adjacent electrode by an electrically resistive film 56. It should be appreciated that the film 56 is less conductive than the electrodes 52. The width of each segment electrode 52 along the sidewall is preferably identical and smaller than the distance between two adjacent longitudinal edges of individual segment electrodes connected by the resistive film 56.

Across the width of the resistive film 56 between adjacent electrodes there is a gradual change in voltage between the electrodes as opposed to a discrete change. As a result, the contact angle between the fluid meniscus and the fluid contact layer 58 gradually changes along the width of the resistive film 56. The contact angle remains constant across the width of the segment electrodes 52. However, the smaller width of the segment electrodes relative to the distance between the adjacent longitudinal edges of individual segment electrodes connected by the resistive film 56 helps to further reduce discontinuous variation of the contact angle along the edge of the fluid. These factors ensure that optical aberrations of the fluid meniscus lens are reduced.

Now referring to Figs. 1 and 5c and with the substitution of the sidewall electrode 2 by this alternative segment electrode configuration, the method of operation is largely similar to that described in the previously described alternative electrode configuration.

Anamorphic fluid meniscus lens shapes can be achieved by combinations of different voltages applied across the segment electrodes 52 and the endwall electrode. The combinations of voltages applied are varied by the ophthalmic optician or the patient using the focal power, cylindrical value and cylindrical axis controls on the manual control unit 27.

Referring to Fig. 6, as described in the previous two embodiments, the exemplary pattern of voltages applied across the segment electrodes may also be applied herein.

The number of electrodes 52 may be any of four or more. The correct angular positioning of the cylindrical axis about the optical axis 50 can be achieved using a lens rotation mechanism operating in a rotational direction 59. Automated mechanical rotation about the optical axis is then conducted in response to operation of the 'cylindrical axis' control on the manual control unit 27. Alternatively, the cylindrical axis orientation may be achieved by rotation about the optical axis 50 of the pattern of voltages applied across the segment electrodes.

In the embodiments of the invention described so far, data relating to the focal power, and other refractive characteristics, of the lens at a set of voltages applied is displayed on the display unit 26. The calibrated value displayed is the result of a predetermined conversion, which is non-linear, between the voltages applied and focal power of the lens. In an alternative envisaged embodiment a predetermined conversion may be made between a measured electrical capacitance and the focal power of the lens. The electrical capacitance of the lens is developed by the contact area of the electrically conducting fluid on the inner surface of the fluid contact layer.

In one embodiment of the present invention, a data display in the form of a liquid crystal display is described for the output of eye deviation data. It is envisaged that alternatives may be used. For example, the data may be fed to a general purpose computer by means of a data communications port and then in turn to a printer. Alternatively, it may also be possible for an electronic message to be created containing the relevant data and sent via a data communications network to a designated recipient, for example the patient.

As a further alternative in a further envisaged embodiment, the data may be fed to a variable fluid meniscus lens manufacture apparatus, as described in our co-pending patent application entitled "Manufacturing Lens Elements" and filed on even date herewith.

This allows corrective lenses, corresponding to the patient's individual eye deviation, to be manufactured. The corrective lenses could be contact lenses or spectacles.

As a yet further envisaged embodiment of the present invention, the use of fluid layers is not limited to the fluids each comprising a liquid. It is possible that one of the fluids may alternatively comprise a gas.

It should be noted that the present invention is not limited to the use of a variable lens of a cylindrical nature as described in this application. It is envisaged that alternative variable lens constructions may be used in the present invention. One such alternative may include the use of a variable lens with frustoconical electrode sidewalls, such as the arrangements described in international patent applications WO 99/18456 and WO 00/58763.

In a further envisaged embodiment of the present invention, one or more non-variable solid lenses may be used in addition to the previously described variable lens. Preferably, the mount for the variable lens includes mountings whereby the solid lens(es) may be positioned such that the optical axis of the solid lens shares the optical axis of the variable lens. The use of additional solid lenses may, for example, achieve a more preferable working point of lens focal power from which the ophthalmic optician could then perform the eye deviation test. Furthermore, the use of additional solid lenses may help reduce any optical aberrations.

In addition, any equivalents and modifications not described above may also be used within the bounds of the scope of the invention, which is defined in the accompanying claims.

## Claims

1. Ophthalmic apparatus for the testing of eye deviation of a patient's eyes, said apparatus comprising:
a) a variable lens having an optical axis and refractive characteristics which cause alterations in direction of rays of light passing through the lens along predetermined incident paths;
b) control means for controlling the refractive characteristics of said variable lens, during the measurement of a patient's eye deviation; and
c) output means for outputting a data value indicative of a measured eye deviation for the patient,
**characterized in that** said variable lens comprises a meniscus , wherein the meniscus separates a layer of a first fluid and a layer of a different, second fluid, and a plurality of electrodes spaced about said optical axis, wherein said control means is adapted to achieve different meniscus shapes by a variation of a pattern of voltages applied across said plurality of electrodes, wherein said meniscus shapes are lens shapes having variable refractive characteristics and including at least approximately spherical or aspherical and at least approximately anamorphic lens shapes.

2. Apparatus according to any preceding claim, wherein said plurality of electrodes comprise one or more pairs of electrodes, and the members of each said pair are located on opposite sides of said optical axis.

3. Apparatus according to any preceding claim, comprising means for rotating the variable lens about the optical axis.

4. Apparatus according to any preceding claim, wherein said control means is adapted to rotate the pattern of applied electrode voltages about the optical axis.

5. Apparatus according to any preceding claim, wherein the plurality of electrodes includes a substantially cylindrical electrode configuration

6. Apparatus according to any preceding claim, wherein said output means is arranged to output a data value to be included in at least part of an ophthalmic prescription to be produced for the patient.

7. Apparatus according to any preceding claim, further comprising a testing object comprising ophthalmic indicia for viewing by a patient during the testing of eye deviation.

8. Apparatus according to any preceding claim, wherein said at least approximately anamorphic lens shapes include at least approximately cylindrical and at least approximately spherocylindrical lens shapes.

9. Apparatus according to any preceding claim, wherein said control means is adapted to provide, in one refractive state, a lens shape having a focal power of a negative value.

10. Apparatus according to any preceding claim, wherein said control means is adapted to provide, in one refractive state, a lens shape having a focal power of a positive value.

11. Apparatus according to any preceding claim, further comprising head mounting means for positioning the variable lens in a desired configuration relative to the patient's eyes.

12. Apparatus according to any preceding claim, further comprising one or more non-variable solid lenses which are positionable so as to share the optical axis of said variable lens.

13. A method of testing the eye deviation of a patient's eyes, said method comprising:
a) providing a variable lens having an optical axis and refractive characteristics which cause alterations in direction of rays of light passing through the lens along predetermined incident paths;
b) controlling the refractive characteristics of the variable lens during the measurement of a patient's eye deviation; and
c) outputting a data value indicative of a measured eye deviation for the patient,
**characterized in that** said provided variable lens comprises a meniscus , wherein the meniscus separates a layer of a first fluid and a layer of a different, second fluid, and a plurality of electrodes spaced about said optical axis, wherein said controlling in step b) involves varying a pattern of voltages applied across said plurality of electrodes to achieve different meniscus shapes, wherein said meniscus shapes are lens shapes having variable refractive characteristics and including at least approximately spherical or aspherical and at least approximately anamorphic lens shapes.

14. A method according to claim 13, wherein during the measurement the patient views a testing object comprising ophthalmic indicia through the variable lens.

15. A method according to claim 13 or 14, wherein the data value indicative of a measured eye deviation for the patient is recorded when the patient can view the ophthalmic indicia at a level of ability at least matching a predetermined threshold of viewing ability.

16. A method according to any of claims 13 to 15, comprising generating ophthalmic prescription data including details of the patient's eye deviation as indicated by said output data value.

## Patentansprüche

1. Ophthalmisches Gerät zur Untersuchung der Augen eines Patienten auf Fehlsichtigkeit, wobei das genannte Gerät Folgendes umfasst:
a) eine variable Linse mit einer optischen Achse und Brechungseigenschaften, die die Richtung der auf vorgegebenen Einfallspfaden durch die Linse fallenden Lichtstrahlen verändern;
b) Mittel zum Steuern der Brechungseigenschaften der genannten variablen Linse während der Messung der Fehlsichtigkeit eines Patienten; und
c) Mittel zum Ausgeben eines Datenwertes, der eine gemessene Fehlsichtigkeit des Patienten angibt;
**dadurch gekennzeichnet, dass** die genannte variable Linse einen Meniskus umfasst, der eine Schicht aus einem ersten Fluid und eine Schicht aus einem anderen, zweiten Fluid voneinander trennt, und eine Vielzahl von Elektroden, die um die genannte optische Achse herum in einem Abstand angeordnet sind, wobei die genannten Steuermittel vorgesehen sind, um unterschiedliche Meniskusformen zu erreichen, indem ein Muster von an die genannte Vielzahl von Elektroden angelegten Spannungen variiert wird, wobei die genannten Meniskusformen Linsenformen mit variablen Brechungseigenschaften sind und mindestens annähernd sphärische oder asphärische und mindestens annähernd anamorphe Linsenformen umfassen.

2. Gerät nach einem der vorhergehenden Ansprüche, wobei die genannte Vielzahl von Elektroden ein oder mehrere Elektrodenpaare umfasst und die Mitglieder jedes genannten Paares sich auf gegenüberliegenden Seiten der genannten optischen Achse befinden.

3. Gerät nach einem der vorhergehenden Ansprüche, mit Mitteln zum Drehen der variablen Linse um die optische Achse.

4. Gerät nach einem der vorhergehenden Ansprüche, wobei die genannten Steuermittel vorgesehen sind, um das Muster der angelegten Elektrodenspannungen um die optische Achse zu drehen.

5. Gerät nach einem der vorhergehenden Ansprüche, wobei die Vielzahl von Elektroden eine im Wesentlichen zylindrische Elektrodenkonfiguration umfasst.

6. Gerät nach einem der vorhergehenden Ansprüche, wobei die genannten Ausgabemittel vorgesehen sind, um einen Datenwert auszugeben, der in mindestens einen Teil eines ophthalmischen Rezepts aufzunehmen ist, das für den Patienten erstellt wird.

7. Gerät nach einem der vorhergehenden Ansprüche, das weiterhin ein Untersuchungsobjekt mit ophthalmischen Indizes zur Betrachtung durch einen Patienten während der Untersuchung auf Fehlsichtigkeit umfasst.

8. Gerät nach einem der vorhergehenden Ansprüche, wobei die genannten mindestens annähernd anamorphen Linsenformen mindestens zylindrische und mindestens annähernd sphärozylindrische Linsenformen umfassen.

9. Gerät nach einem der vorhergehenden Ansprüche, wobei die genannten Steuermittel vorgesehen sind, um in einem Brechungszustand eine Linsenform mit einer Brechkraft mit negativem Wert zu schaffen.

10. Gerät nach einem der vorhergehenden Ansprüche, wobei die genannten Steuermittel vorgesehen sind, um in einem Brechungszustand eine Linsenform mit einer Brechkraft mit positivem Wert zu schaffen.

11. Gerät nach einem der vorhergehenden Ansprüche, das weiterhin am Kopf montierbare Mittel zum Positionieren der variablen Linse in einer gewünschten Konfiguration in Bezug auf die Augen des Patienten umfasst.

12. Gerät nach einem der vorhergehenden Ansprüche, das weiterhin ein oder mehrere nicht-variable feste Linsen umfasst, die so positioniert werden können, dass sie an der optischen Achse der genannten variablen Linse teilhaben.

13. Verfahren zur Untersuchung der Augen eines Patienten auf Fehlsichtigkeit, wobei das genannte Verfahren Folgendes umfasst:
a) Schaffen einer variablen Linse mit einer optischen Achse und Brechungseigenschaften, die die Richtung der auf vorgegebenen Einfallspfaden durch die Linse fallenden Lichtstrahlen verändern;
b) Steuern der Brechungseigenschaften der genannten variablen Linse während der Messung der Fehlsichtigkeit eines Patienten; und
c) Ausgeben eines Datenwertes, der eine gemessene Fehlsichtigkeit des Patienten angibt;
**dadurch gekennzeichnet, dass** die genannte geschaffene variable Linse einen Meniskus umfasst, der eine Schicht aus einem ersten Fluid und eine Schicht aus einem anderen, zweiten Fluid voneinander trennt, und eine Vielzahl von Elektroden, die um die genannte optische Achse herum in einem Abstand angeordnet sind, wobei das genannte Steuern in Schritt b) das Variieren eines Musters von an die genannte Vielzahl von Elektroden angelegten Spannungen umfasst, um verschiedene Meniskusformen zu erreichen, wobei die genannten Meniskusformen Linsenformen mit variablen Brechungseigenschaften sind und mindestens annähernd sphärische oder asphärische und mindestens annähernd anamorphe Linsenformen umfassen.

14. Verfahren nach Anspruch 13, wobei der Patient während der Messung ein Untersuchungsobjekt mit ophthalmischen Indizes durch die variable Linse sieht.

15. Verfahren nach Anspruch 13 oder 14, wobei der Datenwert, der die gemessene Fehlsichtigkeit des Patienten angibt, aufgezeichnet wird, wenn der Patient die ophthalmischen Indizes mit einem Maß an Sehfähigkeit sehen kann, das mindestens einem zuvor festgelegten Schwellenwert der Sehfähigkeit entspricht.

16. Verfahren nach einem der Ansprüche 13 bis 15, das das Erzeugen von ophthalmischen Rezeptdaten umfasst, die Details bezüglich der Fehlsichtigkeit eines Patienten enthalten, wie durch den genannten ausgegebenen Datenwert angegeben.

## Revendications

1. Appareil ophtalmologique pour l'examen de déformations de la vue d'un patient, ledit appareil comprenant :
a) une lentille variable qui a un axe optique et des caractéristiques de réfraction qui sont causes d'altérations dans la direction de rayons de lumière qui passent au travers de la lentille le long de chemins incidents prédéterminés;
b) un moyen de commande pour commander les caractéristiques de réfraction de ladite lentille variable pendant la mesure d'une déformation de la vue d'un patient; et
c) un moyen de sortie pour sortir une valeur de donnée indicative d'une déformation de la vue mesurée pour le patient,
**caractérisé en ce que** ladite lentille variable comprend un ménisque, où le ménisque sépare une couche d'un premier fluide et une couche d'un deuxième fluide différent, et une pluralité d'électrodes espacées autour dudit axe optique, où ledit moyen de commande est approprié pour réaliser différentes formes de ménisque par une variation d'une configuration de tensions appliquées à travers ladite pluralité d'électrodes, où lesdites formes de ménisque sont des formes de lentille qui ont différentes caractéristiques de réfraction variables et comprennent au moins des formes de lentille approximativement sphériques ou asphériques et au moins approximativement anamorphotiques.

2. Appareil selon la revendication précédente, dans lequel ladite pluralité d'électrodes comprend une ou plusieurs paires d'électrodes, et les éléments de chaque paire sont situés sur des côtés opposés dudit axe optique.

3. Appareil selon une quelconque des revendications précédentes, comprenant un moyen pour faire tourner la lentille variable autour de l'axe optique.

4. Appareil selon une quelconque des revendications précédentes, dans lequel ledit moyen de commande est propre à faire tourner la configuration de tensions d'électrodes appliquées autour de l'axe optique.

5. Appareil selon une quelconque des revendications précédentes, dans lequel la pluralité d'électrodes comprend une configuration d'électrodes essentiellement cylindrique.

6. Appareil selon une quelconque des revendications précédentes, dans lequel ledit moyen de sortie est disposé pour générer une valeur de donnée à inclure dans au moins une partie d'une prescription ophtalmologique à produire pour le patient.

7. Appareil selon une quelconque des revendications précédentes, comprenant en outre un objet servant à l'examen qui comporte des signes visuels à regarder par un patient pendant l'examen de la vue.

8. Appareil selon une quelconque des revendications précédentes, dans lequel lesdites formes de lentille au moins approximativement anamorphotiques comprennent des formes de lentille au moins approximativement cylindriques et au moins approximativement sphéro-cylindriques.

9. Appareil selon une quelconque des revendications précédentes, dans lequel ledit moyen de commande est à même, dans un état de réfraction, de procurer une forme de lentille qui a une puissance focale de valeur négative.

10. Appareil selon une quelconque des revendications précédentes, dans lequel ledit moyen de commande est à même, dans un état de réfraction, de procurer une forme de lentille qui a une puissance focale de valeur positive.

11. Appareil selon une quelconque des revendications précédentes, comprenant en outre un moyen de fixation sur la tête pour positionner la lentille variable dans une configuration souhaitée par rapport aux yeux du patient.

12. Appareil selon une quelconque des revendications précédentes, comprenant en outre une ou plusieurs lentilles pleines non variables qui peuvent être positionnées de façon à partager l'axe optique de ladite lentille variable.

13. Procédé d'examen de déformations de la vue d'un patient, ledit procédé comprenant :
a) le fait de disposer une lentille variable qui a un axe optique et des caractéristiques de réfraction qui sont causes d'altérations dans la direction de rayons de lumière qui passent au travers de la lentille le long de chemins incidents prédéterminés;
b) le fait de commander les caractéristiques de réfraction de la lentille variable pendant la mesure d'une déformation de la vue d'un patient; et
c) le fait de générer une valeur de donnée indicative d'une déformation de la vue mesurée pour le patient,
**caractérisé en ce que** ladite lentille variable fournie comprend un ménisque, où le ménisque sépare une couche d'un premier fluide et une couche d'un deuxième fluide différent, et une pluralité d'électrodes espacées autour dudit axe optique, où ledit fait de commander à l'étape b) implique le fait de faire varier une configuration de tensions appliquées aux bornes de ladite pluralité d'électrodes, où lesdites formes de ménisque sont des formes de lentille qui ont différentes caractéristiques de réfraction variables et comprennent au moins des formes de lentille approximativement sphériques ou asphériques et au moins approximativement anamorphotiques.

14. Procédé selon la revendication 13, dans lequel, pendant l'examen, le patient regarde, au travers de la lentille variable, un objet servant à l'examen qui comprend un signe visuel.

15. Procédé selon la revendication 13 ou 14, dans lequel les valeurs de données indicatives d'une déformation de la vue mesurée pour le patient sont enregistrées quand le patient peut voir le signe visuel à un niveau de capacité qui correspond au moins à un seuil prédéterminé de capacité de vision.

16. Procédé selon une quelconque des revendications 13 à 15, comprenant le fait de produire des données de prescription ophtalmologique comprenant des détails sur la déformation de la vue du patient tels qu'indiqués par lesdites valeurs de données générées.
